Europäisches Patentamt

European Patent Office

Office européen des brevets

(19)

(11) Numéro de publication: **0 180 505**
**B1**

(12) **FASCICULE DE BREVET EUROPEEN**

(45) Date de publication du fascicule du brevet: **26.09.90**

(51) Int. Cl.⁵: **A 61 K 7/48**

(21) Numéro de dépôt: **85402002.1**

(22) Date de dépôt: **15.10.85**

(54) **Cosmétique pour retarder le vieillissement de la peau et procédé d'application.**

(30) Priorité: **19.10.84 FR 8416038**
**21.02.85 FR 8502518**

(43) Date de publication de la demande:
**07.05.86 Bulletin 86/19**

(45) Mention de la délivrance du brevet:
**26.09.90 Bulletin 90/39**

(84) Etats contractants désignés:
**CH DE GB IT LI**

(56) Documents cités:
**DE-A-2 019 226**
**FR-A-1 215 850**
**FR-A-1 390 184**
**FR-A-2 163 348**
**FR-A-2 509 989**

(73) Titulaire: **C L A R I N S**
**4, rue Berteaux-Dumas**
**F-92200 Neuilly Sur Seine (FR)**

(72) Inventeur: **Courtin, Olivier**
**7, rue Guénégaud**
**F-75006 Paris (FR)**

(74) Mandataire: **Bezault, Jean et al**
**Cabinet Netter 40, rue Vignon**
**F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un cosmétique pour retarder le vieillissement de la peau, ainsi qu'un procédé pour son application.

On sait que la peau est formée d'un ensemble de cellules dont l'état se réflète dans l'aspect extérieur de l'épiderme. Le processus de vieillissement de la peau résulte d'un ralentissement des fonctions des cellules de la peau, lesquelles fonctions sont essentiellement la nutrition, la respiration, l'hydratation, la régénération et la protection du tissu sousjacent.

Le processus de vieillissement de la peau est un phénomène naturel dont les causes sont variables et qui peut être plus ou moins rapide d'un individu à l'autre. En règle générale, c'est vers la trentaine que les conséquences de ce vieillissement de la peau commencent à apparaître. C'est en effet vers cet âge que débute le ralentissement du fonctionnement de certaines cellules dont le rôle est très important dans le maintien de l'intégrité de la structure de la peau.

Cependant, ce phénomène peut débuter à un âge plus précoce ou au contraire plus avancé, compte tenu de différents facteurs, tels que le patrimoine génétique, l'état général de santé, le mode de vie, qui sont propres à chaque individu.

On connaît des cosmétiques destinés à combattre ou retarder le vieillissement de la peau.

Ces cosmétiques sont d'une efficacité moyenne. Ils contiennent peu de principes actifs et en faible concentration. Le problème de l'accroissement du nombre des principes actifs et également celui de l'augmentation de la concentration de chacun de ceux-ci n'a pas encore été résolu. Les tentatives faites dans ce sens se sont heurtées principalement à l'instabilité du cosmétique, vraisemblablement due à l'incompatibilité entre eux des principes actifs et de leur support.

L'invention résoud ces difficultés d'une manière étonnament simple et permet de retarder ou combattre le vieillissement de la peau avec une efficacité bien supérieure à ce qu'il est possible d'atteindre jusqu'ici.

Elle est basée sur cette constatation que les principes actifs propres à retarder le vieillissement de la peau peuvent être classés en deux catégories, la première comprenant les principes actifs solubles dans l'eau ou hydrosolubles, la seconde les principes actifs solubles dans les huiles ou corps gras ou liposolubles et que les principes actifs d'une seule et même catégorie peuvent coexister en grand nombre dans le même solvant, eau ou corps gras, et chacun d'eux suivant une concentration inaccessible jusqu'ici.

Jusqu' à présent, on s'est efforcé d'une manière générale, dans le domaine des cosmétiques, de fournir à l'utilisateur ou à l'utilisatrice un produit unique pour permettre une application simple comme souhaité d'une manière générale. FR-A-2 509 989 décrit par exemple un tel produit, comprenant une phase huileuse et une phase aqueux. L'invention, prenant le contre-pied de cette coutume, propose un cosmétique constitué par deux compositions distinctes, contenues respectivement dans des réceptacles séparés, l'une constituée par des principes actifs hydrosolubles en solution aqueuse et l'autre par des principes actifs liposolubles en solution grasse.

L'invention prescrit alors l'application sur la peau d'une première composition et l'application successive quasisimultanée de la seconde composition. L'effet obtenu par la présence simultanée sur la peau de principes actifs hydrosolubles et de principes actifs liposolubles suivant des concentrations élevées, comme permises par l'invention, fournit par un effet de synergie inaccessible jusqu'ici des résultats bien supérieurs à ceux qu'on pouvait obtenir antérieurement.

Le cosmétique qui fait l'objet de l'invention est tel que défini dans la revendication 1. Les compositions qui le constituent sont présentées séparément et sont prévues pour être appliquées successivement afin que chacune d'elles exerce sur la peau une action complémentaire de celle de l'autre, compte tenu de la complémentarité technique et biologique des principes actifs contenus dans chacune des compositions. Cette coopération est tout à fait différente de celle décrite dans FR-A-2 163 348. où l'une des phases sert à activer une substance contenue dans l'autre phase. La concentration en au moins un principe actif dans chacune des compositions du cosmétique de l'invention est supérieure à ce qui est connu dans les compositions en émulsion, c'est-à-dire les compositions réunissant simultanément des principes actifs hydrosolubles et des principes actifs liposolubles.

Du fait même que les deux compositions sont présentées séparément, les principes actifs de la première et de la deuxième composition peuvent être présents dans une concentration supérieure à la concentration maximale où ils seraient présents si les deux compositions étaient mélangées au préalable sous forme d'une émulsion.

On a ainsi l'assurance que lorsque les deux compositions selon l'invention sont appliquées sur la peau, les différents principes actifs sont présents à des concentrations respectives suffisamment élevées pour que chacun de ces principes puisse exercer pleinement son activité.

Etant donné qu'il est difficile de déceler ou de prévoir les raisons, du vieillissement de la peau, qui varient d'un individu à l'autre, on a l'assurance que le cosmétique de l'invention permet de retarder ce vieillissement dans tous les cas du fait de la complémentarité des principes actifs utilisés et de leur présence en concentratiors élevées ou surconcentrations.

Les principes actifs présents dans la première et la deuxième composition sont choisis pour leur action contre le ralentissement des fonctions biologiques de la peau.

La première composition comporte au moins un principe actif hydrosolubles choisi parmi les suivants:

Mannuronate de silanol: c'est un produit riche en silicium qui assure la solidité et la rigidité des fibres de la peau entre elles et permet le maintien

de la structure cutanée en bonne condition.

Extrait biologique de rate bovine: c'est un produit qui facilite la respiration des cellules et qui évite donc l'accumulation des toxines au niveau cellulaire.

Extrait biologique de moelle: il agit comme facteur de croissance cellulaire.

Silymarine (extrait végétal de Chardon Marie): il est connu pour ses propriétés anti-inflammatoires et pour ses propriétés protectrices de la membrane cellulaire.

PCA Na: (sel de sodium de l'acide pyrrolidone carboxylique): c'est un facteur naturel d'hydratation.

Panthénol: c'est une substance vitaminique protectrice.

Mucopolysaccharides comme l'acide hyaluronique: ce sont des produits connus pour leurs propriétés hydratantes et régénérantes.

Acides aminés végétaux: ce sont des éléments de base de toutes les synthèses biologiques. Ils agissent également comme facteurs nutritifs.

Extrait végétal d'Echinacée: c'est une plante dont on utilise le rhizome, lequel est riche en huiles essentielles qui agissent en stimulant la croissance cellulaire.

Dans un mode de réalisation préféré de l'invention, tous les principes actifs hydrosolubles cités précédemment sont présents en solution dans un milieu aqueux, lequel est avantageusement une eau florale.

Cette première composition est de préférence réalisée sous la forme d'un gel aqueux ce qui permet une application aisée sur la peau.

La deuxième composition faisant partie de l'ensemble cosmétique selon l'invention comporte au moins un principe actif choisi parmi les suivants:

insaponifiable de soja,

insaponifiable d'avocat,

insaponifiable de karité, les propriétés régénérantes et restructurantes de ces insaponifiables étant bien connues.

beurre de Pentadesma: c'est une substance végétale issue d'un arbre poussant en Afrique, et qui présente un fort pouvoir cicatrisant et protecteur des agressions extérieures.

huile de noix: c'est un produit riche en vitamines.

Dans un mode de réalisation préféré de l'invention, les cinq principes actifs liposolubles ci-dessus sont présents simultanément dans la deuxième composition de l'invention.

Ces cinq principes actifs sont mis en solution dans un milieu huileux approprié absorbable par l'épiderme, de préférence dans une huile.

La présente invention vise également un procédé d'application d'un ensemble cosmétique comprenant une première composition et une deuxième composition telles que définies plus haut. Ce procédé se caractérise essentiellement par le fait que c'est au moment même de l'utilisation que les deux compositions sont réunies, donc sur la peau, et non pas préalablement à l'application, comme c'est le cas avec les produits cosmétiques vendus sous forme d'une émulsion.

Les deux compositions constituant le cosmétique de l'invention sont présentées dans deux réceptacles, flacons ou tubes, séparés à l'intérieur d'un même coffret ou emballage.

Le cosmétique de l'invention est de préférence à utiliser quotidiennement en appliquant successivement la composition comportant les principes actifs liposolubles et ensuite la composition comportant les principes actifs hydrosolubles.

Toutefois, entre également dans le cadre de l'invention le procédé qui consiste à appliquer d'abord la composition contenant les principes actifs hydrosolubles et ensuite la composition comportant les principes actifs liposolubles.

Les deux compositions peuvent être appliquées immédiatement en succession.

En variante, on applique d'abord une des deux compositions puis on attend un certain temps avant d'appliquer la deuxième composition.

Dans un mode de mise en oeuvre du procédé d'application du cosmétique, on verse une quantité convenable de la première composition et une quantité convenable de la seconde composition dans le creux de l'une des mains, on réalise le mélange des deux compositions en frottant les faces internes des mains (paumes et surfaces internes des doigts) l'une contre l'autre et, avec toute la face interne des mains, on applique le cosmétique contre la peau à traiter, qui peut être celle de l'un et l'autre côtés du visage.

Il a été constaté que les compositions respectivement à principe(s) actif(s) hydrosoluble(s) et à principe(s) actif(s) liposoluble(s) formaient, après le frottement des surfaces internes des mains l'une contre l'autre, une émulsion homogène propre à être appliquée sur la peau.

Le cosmétique est ainsi utilisé dans les meilleurs conditions d'efficacité.

D'une part, en effet, il contient tous les principes actifs présents dans l'une et l'autre des compositions, les surfaces internes des mains n'absorbant pratiquement aucun des principes actifs des compositions car elles ne présentent pas de glandes sébacées.

Par ailleurs, la surface interne de chacune des mains est sensiblement égale à celle d'un côté du visage. On obtient donc en une seule opération le traitement souhaité.

D'autre part, le frottement l'une contre l'autre des surfaces internes des mains élève la température du film qui recouvre chacune d'elles.

Dans ces conditions, le film appliqué sur la peau du visage est à une température idéale pour le traitement, n'entraînant aucune contraction des pores de la peau à traiter.

Le cosmétique selon l'invention offre ainsi l'avantage de réunir différents principes actifs à des concentrations suffisamment élevées pour pouvoir agir efficacement et simultanément, ou quasi-simultanément, sur la nutrition cellulaire, la respiration cellulaire, l'hydratation, la régénération, la protection et la défense cellulaire de la peau.

L'invention est en outre illustrée par l'exemple suivant:

5  EP 0 180 505 B1  6

On prépare un cosmétique comprenant une composition aqueuse et une composition grasse contenues chacune dans un réceptacle distinct.

## Composition aqueuse

Elle comprend en mélange dans une base aqueuse, telle qu'un gel aqueux, les principes actifs suivants dans les concentrations indiquées, ces concentrations étant exprimées en rapport poids/poids.

Mannuronate de silanol 3%
Extrait biologique de rate bovine 5%
Extrait biologique de moelle 5%
Silymarine 2%
PCA Na 5%
Panthenol, 0,5%
Mucopolysaccharides 1,5%
Acides aminés végétaux 2%
Extrait végétal d'échinacée 5%

## Composition grasse

Elle comprend en mélange dans une base grasse, telle qu'une huile absorbable par l'épiderme, les principes actifs liposolubles suivants dans les concentrations indiquées, ces concentrations étant exprimées en rapport poids/poids comme pour la composition précédente.

Insaponifiables d'avocat Soja - Karite 3%
Beurre de Pendadesma 1%
Huile de noix 5%

Chacune des deux compositions précédentes peut contenir des additifs ou adjuvants usuels en cosmétologie, comme par exemple des parfums.

## Revendications

1. Cosmétique comprenant une première composition aqueuse et une seconde composition huileuse à mélanger au moment de l'emploi, caractérisé en ce que la première composition contient au moins un principe actif hydrosoluble exerçant une action contre le vieillissement de la peau, choisi parmi le mannuronate de silanol, l'extrait biologique de rate bovine, l'extrait biologique de moelle, la silymarine, le PCA Na (sel de sodium de l'acide pyrrolidone carboxylique) le panthénol, les mucopolysaccharides, les acides aminés végétaux et l'extrait végétal d'Echinacée, en solution dans un milieu aqueux, et que la seconde composition contient au moins un principe actif liposoluble exerçant une action contre le vieillissement de la peau, choisi parmi l'insaponifiable de soja, l'insaponifiable d'avocat, l'insaponifiable de karité, le beurre de pentadesma et l'huile de noix, en solution dans un milieu huileux, chacun de ces principes actifs étant présent dans la composition correspondante à une concentration supérieure à la concentration maximale qu'il peut atteindre lorsque ces deux milieux forment une émulsion.

2. Cosmétique selon la revendication 1, caractérisé en ce que la première composition comprend tous lesdits principes actifs hydrosolubles.

3. Cosmétique selon l'une des revendications 1 et 2, caractérisé en ce que le milieu aqueux de la première composition est une eau florale.

4. Cosmétique selon l'une des revendications précédentes, caractérisé en ce que la première composition se présente sous forme d'un gel.

5. Cosmétique selon l'une des revendications précédentes, caractérisé en ce que la deuxième composition comprend tous lesdits principes actifs liposolubles.

6. Cosmétique selon l'une des revendications précédentes, caractérisé en ce que la deuxième composition se présente sous forme d'une huile absorbable par l'épiderme.

7. Procédé d'application d'un cosmétique selon l'une des revendications précédentes, comprenant une première composition liquide comportant des principes actifs hydrosolubles et une deuxième composition liquide comportant des principes actifs liposolubles, caractérisé en ce que c'est au moment même de l'utilisation que les deux compositions sont réunies sur la peau.

8. Procédé selon la revendication 7, caractérisé en ce qu'une quantité convenable de la première composition et une quantité convenable de la deuxième composition sont versées dans le creux de l'une des mains, en ce qu'on frotte l'une contre l'autre les surfaces internes des mains pour assurer le mélange des compositions et en ce qu'on applique lesdites surfaces internes contre la peau à traiter, qui peut être celle de l'un et/ou l'autre côtés du visage.

9. Composition aqueuse faisant partie d'un cosmétique selon l'une des revendications 1 à 6.

10. Composition huileuse faisant partie d'un cosmétique selon l'une des revendications 1 à 6.

## Patentansprüche

1. Kosmetikum mit einer ersten, wässrigen Lösung und einer zweiten, öligen Lösung, die zum Zeitpunkt der Anwendung zu mischen sind, dadurch gekennzeichnet, daß die erste Lösung wenigstens einem aktiven, wasserlöslichen Stoff, der gegen das Altern der Haut eine Wirkung ausübt und der ausgewählt ist aus dem Silanolmannuronat, dem biologischen Extrakt der Rindermilz, dem biologischen Extrakt des Knochenmarks, dem Silymarin, dem Na-PCA (Natriumsalz der karboxylischen Pyrrolidonsäure), den Panthenol, den Mykopolysacchariden, den pflanzlichen Aminsäuren und dem pflanzlichen Extrakt der Echinazee, in einem wässrigen Milieu gelöst enthält und daß die zweite Lösung wenigstens einen aktiven, fettlöslichen Wirkstoff, der gegen das Altern der Haut ein Wirkung ausübt und der ausgewählt ist aus dem Nichtverseifbaren der Soja, dem Nichtverseifbaren der Avocado, dem Nichtverseifbaren des Kariten, der Pentadesmabutter und dem Nußöl, in einem fettigen Milieu gelöst enthält, wobei jeder dieser Wirkstoffe in der entsprechenden Lösung in einer Konzentration vorhanden ist, die größer ist als die maximale Konzentration, die er erreichen kann, wenn diese beiden Lösungen eine Emulsion bilden.

2. Kosmetikum nach Anspruch 1, dadurch

4

gekennzeichnet, daß die erste Lösung alle diese wasserlöslichen Wirkstoffe umfaßt.

3. Kosmetikum nach einem der Ansprüche 1 und 2, dadurch gekennzeichnet, daß das wässrige Milieu der ersten Lösung ein Blumenwasser ist.

4. Kosmetikum nach einem der vorhergehenden Ansprüche, dadurch gekennzeichnet, daß sich die erste Lösung in der Form eines Gels darstellt.

5. Kosmetikum nach einem der vorhergehenden Ansprüche, dadurch gekennzeichnet, daß die zweite Lösung alle diese fettlöslichen Wirkstoffe umfaßt.

6. Kosmetikum nach einem der vorhergehenden Ansprüche, dadurch gekennzeichnet, daß die Lösung sich in der Form eines durch die Haut absorbierbaren Öls darstellt.

7. Verfahren zur Anwendung eines Kosmetikums nach einem der vorhergehenden Ansprüche mit einer ersten flüssigen Lösung, die wasserlösliche Wirkstoffe umfaßt, und einer zweiten flüssigen Lösung, die fettlösliche Wirkstoffe umfaßt, dadurch gekennzeichnet, daß die beiden Lösungen im Moment ihrer Verwendung auf der Haut zusammengebracht werden.

8. Verfahren nach Anspruch 7, dadurch gekennzeichnet, daß eine passende Menge der ersten Lösung und eine passende Menge der zweiten Lösung in die hohle handfläche einer Hand gegossen wird, daß man die Handinnenflächen gegeneinander reibt, um die Vermischung der Lösungen sicherzustellen, und daß man die Innenflächen auf die zu behandelnde Haut anwendet, die diejenige der einen und/oder der anderen Gesichtsseite sein kann.

9. Wässrige Lösung, die Bestandteil eines Kosmetikums nach einem der Ansprüche 1 bis 6 ist.

10. Ölige Lösung, die Bestandteil eines Kosmetikums nach einem der Ansprüche 1 bis 6 ist.

**Claims**

1. Cosmetic comprising a first, aqueous composition and a second, oily composition to be mixed at the time of use, characterized in that the first composition contains at least one water-soluble active principle exerting an action against skin ageing, selected from silanol mannuronate, biological extract of bovine spleen, biological extract of bone marrow, silymarin, PCA Na (sodium salt of pyrrolidonecarboxylic acid), dex-panthenol, mucopolysaccharides, plant amino acids and plant extract of Echinacea, in solution in an aqueous medium, and in that the second composition contains at least one fat-soluble active principle exerting an action against skin ageing, selected from soybean unsaponifiable matter, avocado unsaponifiable matter, shea unsaponifiable matter, pentadesma butter and walnut oil, in solution in an oily medium, each of these active principles being present in the corresponding composition at a higher concentration than the maximum concentration which it can attain when these two media form an emulsion.

2. Cosmetic according to Claim 1, characterized in that the first composition comprises all the said water soluble active principles.

3. Cosmetic according to one of Claims 1 and 2, characterized in that the aqueous medium of the first composition is a flower water.

4. Cosmetic according to one of the preceding claims, characterized in that the first composition is presented in the form of a gel.

5. Cosmetic according to one of the preceding claims, characterized in that the second composition comprises all the said fat-soluble active principles.

6. Cosmetic according to one of the preceding claims, characterized in that the second composition is presented in the form of an oil absorbable by the epidermis.

7. Process for applying a cosmetic according to one of the preceding claims, comprising a first liquid composition containing water-soluble active principles and a second liquid composition containing fat-soluble active principles, characterized in that the two compositions are combined on the skin at the actual time of use.

8. Process according to Claim 7, characterized in that a suitable amount of the first composition and a suitable amount of the second composition are poured into the hollow of one hand, in that the inner surfaces of the hands are rubbed against one another to provide for mixing of the compositions and in that the said inner surfaces are applied against the skin to be treated, which can be that of either or both sides of the face.

9. Aqueous composition forming part of a cosmetic according to one of claims 1 to 6.

10. Oily composition forming part of a cosmetic according to one of Claims 1 to 6.